# EUROPÄISCHE PATENTSCHRIFT

(11) **EP 2 132 162 B1**
(45) Veröffentlichungstag und Bekanntmachung des Hinweises auf die Patenterteilung: **22.06.2011**
(21) Anmeldenummer: 08709244.1
(22) Anmeldetag: 28.02.2008
(51) Int. Cl.: C07C 209/48, C07C 253/30

(54) **VERFAHREN ZUR HERSTELLUNG VON ETHYLENAMINEN**
METHOD FOR PRODUCING ETHYLENEAMINES
PROCÉDÉ DE PRÉPARATION D'ÉTHYLÈNE AMINES

(30) Priorität: 01.03.2007 EP 07103293
(43) Veröffentlichungstag der Anmeldung: 16.12.2009
(73) Patentinhaber: BASF SE, 67056 Ludwigshafen (DE)
(72) Erfinder: OFTRING, Alfred, 67098 Bad Duerkheim (DE); DAHMEN, Kirsten, 67251 Freinsheim (DE); HAHN, Thilo, 67292 Kirchheimbolanden (DE); HUGO, Randolf, 67246 Dirmstein (DE); BAUMANN, Katrin, 68529 Mannheim (DE); MELDER, Johann-Peter, 67459 Boehl-Iggelheim (DE)
(86) Internationale Anmeldenummer: PCT/EP2008/052414
(87) Internationale Veröffentlichungsnummer: WO 2008/104583

(56) Entgegenhaltungen:
- EP-A- 1 209 146

## Beschreibung

Die Erfindung betrifft ein Verfahren zur Herstellung eines Ethylenamingemisches durch Hydrierung eines Aminonitrilgemisches an einem Katalysator. Aus dem erhaltenen Ethylenamingemisch können die einzelnen Ethylenamine gegebenenfalls isoliert werden.

Es ist generell bekannt, dass Nitrile in Gegenwart von Katalysatoren zu den entsprechenden Aminen hydriert werden können. In Abhängigkeit von den gewählten Reaktionsparametern liefern die bekannten Verfahren die gewünschten Produkte, beispielsweise primäre Amine als Hauptprodukt und sekundäre und tertiäre Amine als Nebenprodukte. Problematisch ist hierbei oft, dass das gewünschte Produkt mit niedriger Selektivität und/oder niedriger Ausbeute anfällt, häufig auch begleitet von einer raschen Inaktivierung des verwendeten Katalysators.

Für Verfahren zur Herstellung von Aminen durch Hydrierung von Nitrilen ist zudem bekannt, dass ein gewisser Anteil an Ammoniak die Selektivität der Hydrierung zu primären Aminen begünstigt, und die Bildung von sekundären und tertiären Aminen unterdrückt. Allerdings verursacht die Hydrierung in Gegenwart von Ammoniak zusätzlichen technischen Aufwand, der mit der Abtrennung aus dem Produktstrom, der Aufarbeitung und der eventuellen Rückführung des Ammoniaks verbunden ist. Darüber hinaus können höhere Drücke bei der Hydrierung erforderlich sein, da der Partialdruck des Ammoniaks berücksichtigt werden muss.

So kann durch Hydrierung von Aminoacetonitril (AAN) als Hauptprodukt Ethylendiamin (EDA) hergestellt werden, welches ein Ausgangsstoff beispielsweise für die Synthese von Komplexbildnern oder Bleichaktivatoren ist, die unter anderem als Waschmittel-oder Reinigungsadditive Verwendung finden. Analog liefert die Hydrierung von Iminodiacetonitril (IDAN) als Hauptprodukt Diethylentriamin (DETA). Jedoch fallen bei der Hydrierung von AAN bzw. IDAN immer auch DETA bzw. EDA als Nebenprodukte an. Je nach den gewählten Reaktionsbedingungen können auch weitere Aminverbindungen als Nebenprodukte anfallen.

In DE-A 3 003 729 wird ein Verfahren zum Hydrieren von aliphatischen Nitrilen, Alkylenoxynitrilen und Alkylenaminonitrilen zu primären Aminen in Anwesenheit eines Lösungsmittelsystems an einem Kobalt- oder Rutheniumkatalysator beschrieben. Das verwendete Lösungsmittelsystem enthält neben Wasser und Ammoniak einen Ether oder Polyether, mit vorzugsweise 4 bis 6 Kohlenstoffatomen, und einem Kohlenstoff zu Sauerstoffverhältnis von 2 : 1 bis 5 : 1, wie Dioxan, Tetrahydrofuran, Methylenglykoldimethylether oder Diethylenglykoldimethylether, wobei cyclische Ether wie Dioxan und Tetrahydrofuran besonders bevorzugt sind. Als Nitrilkomponente sind Dinitrile besonders bevorzugt. DE-A 3003 729 offenbart hingegen nicht, dass auch Verbindungen, die sowohl eine Cyanogruppe als auch eine Aminogruppe aufweisen, wie AAN in dem Verfahren verwendet werden können.

EP-A 0 382 508 beschreibt ein Verfahren zur chargenweisen Herstellung von nicht-cyclischen, aliphatischen Polyaminen durch Hydrierung von nicht-cyclischen, aliphatischen Polynitrilen in flüssiger Phase an Raney-Kobalt-Katalysatoren, vorzugsweise in Gegenwart von wasserfreiem Ammoniak. Hierbei wird eine Polynitrillösung einer Reaktionszone zugeführt, die den Raney-Kobalt-Katalysator in einer im wesentlichen sauerstofffreien Atmosphäre enthält. Während des gesamten Reaktionszeitraums wird die Polynitrillösung mit einer Rate zugeführt, die nicht größer ist als die maximale Rate, mit der das Polynitril mit dem Wasserstoff in der Reaktionszone reagiert. Es wird weiterhin ein Reaktionsparameter K benannt, welcher zur Bestimmung der volumetrischen Zufuhrrate geeignet ist. Das beschriebene Verfahren beschränkt sich auf die Herstellung von Polyaminen aus Polynitrilen, wie Iminodiacetonitril (IDAN), Nitrilotriacetonitril oder weitere Verbindungen mit 2 oder mehr Cyanogruppen. Die Umsetzung von Verbindungen mit einer Cyanogruppe, wie AAN zu EDA, wird jedoch nicht beschrieben.

EP-A 212 986 betrifft ein weiteres Verfahren, bei dem aliphatische Polynitrile in Gegenwart eines im Eduktstrom enthaltenen, flüssigen primären oder sekundären Amines an einem granularen Raney-Kobalt-Katalysators zu den entsprechenden Polyaminen hydriert werden können. Unter anderem ist die zwingend vorhandene Aminokomponente EDA neben zahlreichen weiteren primären oder sekundären Aminen aufgeführt. Weiterhin offenbart dieses Dokument konkret, dass IDAN zu DETA hydriert werden kann.

DE-A 1 154 121 betrifft ein Verfahren zur Herstellung von Ethylendiamin, wobei die Edukte Blausäure, Formaldehyd, Ammoniak und Wasserstoff in Gegenwart eines Katalysators in einem so genannten Eintopf-Verfahren zur Reaktion gebracht werden. Sowohl der Ammoniak als auch der Wasserstoff werden im molaren Überschuss gegenüber den in äquimolaren Mengen vorliegenden weiteren Edukten Blausäure und Formaldehyd eingesetzt. In diesem Verfahren wird also das in situ gebildete AAN nicht isoliert, sondern direkt mit Wasserstoff weiter umgesetzt. Nachteilig an diesem Verfahren ist, dass das gewünschte Produkt (EDA) relativ unselektiv in kleinen Mengen anfällt.

US-A 3,255,248 beschreibt ein Verfahren zur Hydrierung von organischen Stickstoff-Kohlenstoff-Verbindungen, die vorzugsweise Nitro-, N-Nitroso-, Isonitroso-, Cyano-oder mit Aromaten substituierte Aminogruppen aufweisen, zu den entsprechenden Aminen in flüssiger Phase unter Verwendung eines gesinterten Katalysators aus Kobalt oder Nickel. Hierbei wird das Edukt entweder alleine oder in Gegenwart eines Lösungsmittels, wie beispielsweise Wasser, Tetrahydrofuran, Methanol, Ammoniak oder das gebildete Reaktionsprodukt, gemeinsam mit dem Wasserstoff auf den Katalysator hinuntergerieselt. Sofern am Stickstoffatom ungesättigte Verbindungen, wie Cyanogruppen hydriert werden, wird die Gegenwart von Ammoniak bei der Umsetzung empfohlen. Dies wird in Beispiel 1 dieses Patents verdeutlicht, wo Aminoacetonitril in Form einer wässrigen Lösung mit flüssigem Ammoniak, aber ohne sonstiges Lösungsmittel auf den gesinterten Katalysator gerieselt wird. Der verwendete Druck lag bei 280atm.

EP-A 1 209 146 betrifft ein weiteres Verfahren zur kontinuierlichen Hydrierung von Nitrilen zu primären Aminen, wobei die jeweiligen Nitrile in flüssiger Phase an einem suspendierten, aktivierten Raney-Katalysator auf Basis einer Legierung aus Aluminium eingesetzt werden und die Reaktion in Abwesenheit von Ammoniak und basischen Alkali- oder Erdalkaliverbindungen durchgeführt wird. Als Nitrile können neben vielen anderen auch AAN sowie IDAN zu den entsprechenden Ethylenaminen umgesetzt werden. Gegebenenfalls kann das zu hydrierende Nitril auch in einem organischen Lösungsmittel gelöst vorliegen, wobei vorzugsweise Alkohole, Amine, Amide, insbesondere N-Methylpyrrolidon (NMP) und Dimethylformamid (DMF), sowie Ether oder Ester eingesetzt werden. In EP-A 1 209 146 finden sich jedoch keine Angaben, dass IDAN und AAN gemeinsam hydriert werden können.

Im Stand der Technik wird somit nirgendwo beschrieben, dass auch Gemische von Aminonitrilen, die IDAN und AAN enthalten, hydriert werden können. Die Verfahren gemäß dem Stand der Technik beschränken sich vielmehr auf die Hydrierung von einzelnen Substanzen.

Aufgabe der vorliegenden Erfindung ist es demnach, ein einfaches und kostengünstiges Verfahren zur Herstellung der Ethylenamine EDA und/oder DETA sowie gegebenenfalls weiterer Ethylenamine wie Pip durch Hydrierung der entsprechenden Aminonitrile bereitzustellen. Dabei soll ein hoher Umsatz bei jeweiliger hoher Selektivität erzielt werden, wobei das Verhältnis von DETA zu EDA variabel ist.

Die Aufgabe wird gelöst durch ein Verfahren zur Herstellung eines Ethylenamingemisches, wobei ein Aminonitrilgemisch enthaltend mindestens 30 Gew.-% Aminoacetonitril (AAN) und mind. 5 Gew.-% Iminodiacetonitril (IDAN) in Gegenwart eines Katalysators hydriert wird. Hydrieren im Rahmen der vorliegenden Erfindung bedeutet die Reaktion des Aminonitrilgemisches mit Wasserstoff.

Das erfindungsgemäße Verfahren hat den Vorteil, dass die Hauptkomponenten des Ethylenamingemisches (EDA und DETA) bei hohem Umsatz und/oder Selektivität hergestellt werden können (höhere Raum-Zeit-Ausbeute). Vorzugsweise wird das eingesetzte Aminonitrilgemisch vollständig oder nahezu vollständig umgesetzt. Dies ist insbesondere bei großtechnischen Verfahren von Bedeutung, da nicht umgesetztes Edukt in der Regel in den Produktionskreislauf zurückgeführt wird bzw. entsorgt werden muss. Verfahren, bei denen größere Mengen an AAN und/oder IDAN nicht umgesetzt werden, sind aufgrund der hohen Instabilität des AAN bzw. IDAN von besonderem Nachteil. Einerseits neigen sowohl AAN als auch IDAN sich bei höheren Temperaturen zu zersetzen, so dass die Zersetzungsprodukte nicht in den jeweiligen Kreislauf zurückgeführt werden können, andererseits kann diese Zersetzung auch mit explosionsartiger Heftigkeit verlaufen. Da im erfindungsgemäßen Verfahren das Aminonitrilgemisch vollständig umgesetzt werden kann, müssen hinsichtlich dessen Rückführung in den Produktionszyklus keine Anstrengungen unternommen werden.

Ein Vorteil der Herstellung eines Ethylenamingemisches - anstelle der Herstellung der einzelnen Komponenten in separaten Kampagnen bzw. in getrennten Verfahren - ist, dass auf die Zudosierung von Ammoniak verzichtet werden kann. Bei der gezielten Herstellung von Ethylenaminen gemäß Stand der Technik werden in der Regel Ammoniak oder andere Additive zur Unterdrückung von sekundären Aminen hinzugefügt. Bei der erfindungsgemäßen Synthese von Ethylenamingemischen ist die Unterdrückung der Dimerisierung nicht notwendig, da die Dimere im Produktmix anfallen und Wertprodukte darstellen. In getrennten Synthesen dagegen bedeuten in kleinen Konzentrationen anfallende Komponenten Trennprobleme, und stören daher, selbst wenn sie Wertprodukte darstellen. Verzicht von Ammoniak bedeutet apparative Einsparungen infolge fehlender Ammoniak-Abtrennung, Lagerung oder Rückführung sowie mögliche geringere Drücke im Hydrierreaktor durch den Wegfall des Eigendrucks des Ammoniaks. Auch aus Sicherheitsgründen ist der Verzicht auf Ammoniak von Vorteil.

Trotz der Tatsache, dass im erfindungsgemäßen Verfahren prinzipiell ein Ethylenamingemisch anfällt, können durch kontinuierliche Isolierung die Hauptkomponenten EDA, DETA und gegebenenfalls auch andere, als Nebenprodukte anfallende Ethylenamine in einer einzigen Anlage erhalten werden. Bei herkömmlichen Verfahren, bei denen jeweils die Aminonitrile separat hydriert werden, fallen prinzipiell immer DETA, EDA bzw. weitere Ethylenamine (jeweils abhängig vom verwendeten Edukt) als Nebenprodukte an. Demzufolge sind im Anschluss an die jeweiligen gezielten Ethylenamin-Synthesen generell die gleichen Aufreinigungsschritte wie im erfindungsgemäßen Verfahren zur Abtrennung der Nebenprodukte vom entsprechenden Hauptprodukt erforderlich. Verfahren zur Abtrennung der bei den Einzelverfahren jeweils anfallenden Nebenprodukte (DETA bzw. EDA) unterscheiden sich somit prinzipiell nicht von Verfahren zur Isolierung der im erfindungsgemäßen Verfahren anfallenden Hauptprodukte (z.B. EDA und DETA), lediglich die abzutrennende Menge an EDA bzw. DETA ist verschieden. Bei Kampagnenfahrweise kommt zudem auch nur die diskontinuierliche Fahrweise in Frage, was aufgrund der gewünschten Mengen unpraktikabel ist. Bei kontinuierlicher Fahrweise sind Abstellungen und Umstellungen der Anlagen in Kauf zu nehmen (Reduzierung der Anlagenverfügbarkeit, Reinigungsaufwand, Produktverlust, Personeller Aufwand etc.). Auch müssen für die Bedürfnisse des Marktes entsprechende Lagerkapazitäten vorhanden sein.

Ebenso ist es als Vorteil anzusehen, dass, je nach Bedürfnissen des Marktes, ein höherer oder niedriger Anteil an EDA bzw. DETA hergestellt werden kann. So können im erfindungsgemäßen Verfahren spezielle Aminonitrilgemisch-Zusammensetzungen gezielt eingesetzt werden, um die im Markt gewünschten Mengenverhältnisse zu bedienen. Das erfindungsgemäße Verfahren liefert bei hoher Selektivität ein Ethylenamingemisch, das mindestens 30 % EDA, neben mindestens 5 % DETA enthält. Auch gegebenenfalls anfallende weitere Ethylenamine können Wertprodukte darstellen und als solche isoliert werden.

Während es sich bei AAN bei Raumtemperatur um eine Flüssigkeit handelt, ist IDAN bei Raumtemperatur (RT) fest und in gängigen inerten Lösemitteln schlecht löslich. Aufgrund der guten Löslichkeit von IDAN in AAN ist im erfindungsgemäßen Verfahren die Handhabung von Feststoffen vermeidbar. Zum Beispiel sind bei Raumtemperatur nur ~10% IDAN in THF löslich, während in AAN gelöst Konzentrationen bis zu 35 % möglich sind.

Ein weiterer Nachteil der separaten Herstellung von DETA ist dessen hohe Komplexierung des Katalysators. Die daraus resultierende Produkthemmung hat eine relativ langsame Hydriergeschwindigkeit zur Folge. Bei der Herstellung von EDA ist die Produkthemmung deutlich geringer, so dass eine erheblich höhere Hydriergeschwindigkeit von AAN, vermutlich wegen niedrigerer Komplexkonstanten, möglich ist. Sofern die Hydrierung in Gegenwart von mindestens 30 Gew.-% AAN wie im erfindungsgemäßen Verfahren durchgeführt wird, ist die Produkthemmung der entsprechenden Ethylenamine reduziert, wobei die Gesamt-Produkthemmung deutlich geringer bzw. nicht mehr feststellbar ist. Für ein gegebenes Aminonitril-Gemisch ist somit die Raum-Zeit-Ausbeute der jeweiligen Komponenten größer als bei der entsprechenden Hydrierung der Einzelkomponenten, bzw. die Hydrierung des Gemisches kann bei deutlich niedrigeren Drücken durchgeführt werden, wodurch deutlich geringere Investitionskosten möglich werden.

Das erfindungsgemäße Verfahren geht von einem Aminonitrilgemisch als Edukt aus. Das Aminonitrilgemisch enthält neben mindestens 30 Gew.-% Aminoacetonitril (AAN) mindestens 5 Gew.-% Iminodiacetonitril (IDAN). Gegebenenfalls können weitere Aminonitrile enthalten sein. AAN ist normalerweise zu 30 bis 95 Gew.-%, bevorzugt zu 50 bis 95 Gew.-%, besonders bevorzugt zu 75 bis 90 Gew.-% im Aminonitrilgemisch enthalten. Das Aminonitrilgemisch enthält IDAN normalerweise zu 5 bis 70 Gew.-%, bevorzugt zu 5 bis 50 Gew.-%. Besonders bevorzugt enthält es IDAN zu 10 bis 25 Gew.-%. Die vorstehenden Gewichtsprozentangaben von AAN und IDAN beziehen sich auf die Gesamtmenge der im Gemisch enthaltenen Aminonitrile. Gegebenenfalls vorhandenes Wasser oder Lösungsmittel sind bei diesen Mengenangaben nicht berücksichtigt.

Generell kann jede Art/Qualität von AAN oder IDAN eingesetzt werden. Gegebenenfalls können die Aminonitrile jeweils auch in Form ihrer wässrigen oder wässrigen ammoniakalischen Lösung eingesetzt werden. Verfahren zur Herstellung AAN oder IDAN sind dem Fachmann bekannt. Vorzugsweise werden AAN und/oder IDAN durch Umsetzung von NH₃ und Formaldehydcyanhydrin (FACH) hergestellt.

IDAN und AAN können getrennt voneinander synthetisiert und vor dem Einsatz im erfindungsgemäßen Verfahren in den entsprechenden Mengen zum Aminonitrilgemisch vereint werden. Gegebenenfalls können AAN und IDAN auch gemeinsam hergestellt werden, das Aminonitrilgemisch enthaltend mindestens 30 Gew.-% AAN und mindestens 5 Gew.-% IDAN lässt sich anschließend durch entsprechendes Auf- und/oder Abkonzentrieren an IDAN bzw. AAN herstellen.

Die beiden Hauptkomponenten des Aminonitrilgemisches sind wie vorstehend bereits erwähnt AAN und IDAN. IDAN ist bei Raumtemperatur ein Feststoff, während AAN eine Flüssigkeit ist, wobei sich IDAN größtenteils in AAN löst. Vorzugsweise wird im erfindungsgemäßen Verfahren das Aminonitrilgemisch selbst als Flüssigkeit (Lösung) der Hydrierung unterzogen. Da das Aminonitrilgemisch bei dem im erfindungsgemäßen Verfahren angewendeten Reaktionsbedingungen als Flüssigkeit der Hydrierung zugefahren werden kann, ist es nicht zwingend erforderlich, dass die Hydrierung des Aminonitrilgemisches in Gegenwart eines weiteren Lösungsmittels, wie z.B. eines organischen Lösungsmittels und/oder Wasser, durchgeführt wird. Die zusätzliche Verwendung eines organischen Lösungsmittels (inerten organischen Verbindung) und/oder von Wasser erweist sich jedoch als vorteilhaft, da insbesondere durch die Verwendung eines organischen Lösungsmittels eine Stabilisierung der einzelnen Komponenten des Aminonitrilgemisches, insbesondere in Gegenwart der resultierenden Amine, erzielt werden kann. Außerdem lässt sich durch die Verwendung von Lösungsmitteln ein Spüleffekt an dem eingesetzten Katalysator erzielen, wodurch dessen Standzeit erhöht bzw. dessen Verbrauch erniedrigt sowie die Katalysatorbelastung verbessert werden kann.

Ein geeignetes Lösungsmittel, welches ein oder mehrere Komponenten umfassen kann, sollte bevorzugt folgende Eigenschaften aufweisen:
(a) das Lösungsmittel sollte stabilisierend auf die Komponenten des Aminonitrilgemisches wirken, insbesondere eine Zersetzung von AAN oder IDAN bei den herrschenden Temperaturen reduzieren;
(b) das Lösungsmittel sollte eine gute Wasserstofflöslichkeit zeigen;
(c) das Lösungsmittel sollte bei den Reaktionsbedingungen inert sein;
(d) das Reaktionsgemisch (Aminonitrilgemisch; gegebenenfalls Wasser sowie Lösungsmittel) sollte unter Reaktionsbedingungen einphasig sein;
(e) die Lösemittelauswahl sollte im Hinblick auf eine bevorzugt destillative Abtrennung des Produktes im Anschluss an die Hydrierung aus dem Produktstrom erfolgen. Wobei energetisch oder apparativ aufwändige Trennungen (z.B. engsiedende Gemische oder schwierig zu trennende Azeotrope) zu vermeiden sind.
(f) das Lösungsmittel sollte gut von den Produkten abtrennbar sein, d.h. die Siedetemperatur sollte sich hinreichend von der der Produkte unterscheiden. Hierbei wird eine niedrigere Siedetemperatur als die der Produkte bevorzugt.

Mögliche Lösungsmittel sind organische Lösungsmittel, beispielsweise Amide, wie N-Methylpyrrolidon (NMP) und Dimethylformamid (DMF), aromatische und aliphatische Kohlenwasserstoffe, wie Benzol und Xylol, Alkohole wie Methanol, Ethanol, Propanol, Isopropanol, Butanol, Isobutanol, sekundäres Butanol und tertiäres Butanol, Amine wie Alkylamine, Ethylenamine, Ester, wie Essigsäuremethylester oder Essigsäureethylester und Ether, wie Diisopropylether, Diisobutylether, Glykoldimethylether, Diglykoldimethylether, Dioxan und Tetrahydrofuran (THF). Bevorzugt werden im erfindungsgemäßen Verfahren Ether verwendet, mehr bevorzugt cyclische Ether und besonders bevorzugt Tetrahydrofuran. In einer weiteren bevorzugten Ausführungsform werden Alkohole, insbesondere Methanol, als organisches Lösungsmittel verwendet.

Das Lösungsmittel wird im Gewichtsverhältnis zu dem eingesetzten Aminonitrilgemisch von 0,1 : 1 bis 15 : 1 eingesetzt. Die Konzentration des Aminonitrilgemisches in der Lösung, in der die Hydrierung durchgeführt wird, sollte so gewählt werden, dass eine geeignete Zuführrate bzw. Verweilzeit eingestellt werden kann. Es ist bevorzugt, das Aminonitrilgemisch zu 10 bis 50 Gew.-% mit dem Lösungsmittel zu vermischen. Bezogen auf die besonders bevorzugten Lösungsmittel Methanol bzw. Tetrahydrofuran ist es beispielsweise vorteilhaft, das Aminonitrilgemisch zu 20 bis 40 Gew.-% bezogen auf das Lösungsmittel einzusetzen.

Die für die Herstellung von Ethylenaminen durch Hydrierung des Aminonitrilgemisches eingesetzte Lösung kann neben dem Aminonitrilgemisch und gegebenenfalls dem Lösungsmittel auch einen Anteil an Wasser enthalten. Bevorzugt wird das Aminonitrilgemisch direkt hydriert, ggf. kann Wasser zumindest teilweise oder ganz abgetrennt und das Aminonitrilgemisch dann hydriert werden.

Gegebenenfalls können in der Lösung, die der Hydrierung durchgeführt wird, zusätzliche Additive enthalten sein. Als Additive kommen prinzipiell Hydroxide wie Alkalimetallhydroxide, Alkoholate, Amide oder Amine wie Ammoniak in Frage. Weiterhin können auch saure Additive, wie zum Beispiel Silikate zusätzlich in der Lösung enthalten sein. Diese Substanzen können als Reinstoff oder gelöst in einem Lösungsmittel zugesetzt werden. Vorzugsweise wird das erfindungsgemäße Verfahren ohne den Zusatz von Additiven durchgeführt.

In einer bevorzugten Ausführungsform des Verfahrens wird der Lösung, in der die Hydrierung durchgeführt wird, kein Ammoniak zugesetzt. Sofern noch Ammoniak in den Edukten bzw. in der gegebenenfalls eingesetzten wässrigen Lösung gelöst ist bzw. als Nebenprodukt bei der Hydrierung freigesetzt wird, ist dies nicht störend. Gegebenenfalls vorhandener Ammoniak kann nach dem Fachmann bekannten Methoden, beispielsweise destillativ, entfernt werden.

Als Katalysatoren zur Hydrierung der Nitril-Funktion zum Amin können Katalysatoren eingesetzt werden, die als aktive Spezies ein oder mehrere Elemente der 8. Nebengruppe des Periodensystems (Fe, Co, Ni, Ru, Rh, Pd, Os, Ir, Pt), bevorzugt Fe, Co, Ni, Ru oder Rh, besonders bevorzugt Co oder Ni enthalten. Darin eingeschlossen sind so genannte Skelett-Katalysatoren (auch als Raney®-Typ bezeichnet; nachfolgend auch: Raney-Katalysator), die durch Auslaugen (Aktivierung) einer Legierung aus hydrieraktivem Metall und einer weiteren Komponente (bevorzugt Al) erhalten werden. Die Katalysatoren können zusätzlich einen oder mehrere Promotoren enthalten. In einer bevorzugten Ausführungsform werden im erfindungsgemäßen Verfahren Raney-Katalysatoren eingesetzt, bevorzugt Raney-Kobalt- oder Raney-Nickel-Katalysatoren und besonders bevorzugt mit mindestens einem der Elemente Cr, Ni oder Fe dotierte Raney-Kobalt- oder mit einem der Elemente Mo, Cr oder Fe dotierte Raney-Nickel-Katalysatoren.

Die Katalysatoren können als Vollkatalysatoren oder geträgert eingesetzt werden. Als Träger kommen bevorzugt Metalloxide wie Al₂O₃, SiO₂, ZrO₂, TiO₂, Gemische von Metalloxiden oder Kohlenstoff (Aktivkohlen, Ruße, Graphit) zur Anwendung.

Die oxidischen Katalysatoren werden vor dem Einsatz außerhalb des Reaktors oder im Reaktor durch Reduktion der Metalloxide in einem Wasserstoff-enthaltendem Gasstrom bei erhöhter Temperatur aktiviert. Wenn die Katalysatoren außerhalb des Reaktors reduziert werden, kann danach eine Passivierung durch einen Sauerstoffenthaltenden Gasstrom oder die Einbettung in ein inertes Material erfolgen, um eine unkontrollierte Oxidation an Luft zu vermeiden und einen sicheren Umgang zu ermöglichen. Als inertes Material können organische Lösungsmittel wie Alkohole aber auch Wasser oder ein Amin, bevorzugt das Reaktionsprodukt, verwendet werden. Eine Ausnahme bei der Aktivierung stellen die Skelett-Katalysatoren dar, die durch Laugung mit wässriger Base, wie z. B. in EP-A 1 209 146 beschrieben, aktiviert werden können.

Je nach durchgeführtem Verfahren (Suspensionshydrierung, Wirbelschichtverfahren, Festbetthydrierung) werden die Katalysatoren als Pulver, Splitt oder Formkörper (bevorzugt Extrudate oder Tabletten) eingesetzt.

Besonders bevorzugte Festbettkatalysatoren sind die in EP-A1 742 045 offenbarten Cobalt-Vollkontakte, dotiert mit Mn, P, und Alkalimetall (Li, Na, K, Rb, Cs). Die katalytisch aktive Masse dieser Katalysatoren besteht vor der Reduktion mit Wasserstoff aus 55 bis 98 Gew.-%, insbesondere 75 bis 95 Gew.-%, Cobalt, 0,2 bis 15 Gew.-% Phosphor, 0,2 bis 15 Gew.-% Mangan und 0,05 bis 5 Gew.-% Alkalimetall, insbesondere Natrium, jeweils berechnet als Oxid.

Weitere geeignete Katalysatoren sind die in EP-A 963 975 offenbarten Katalysatoren, deren katalytisch aktive Masse vor der Behandlung mit Wasserstoff 22 bis 40 Gew.-% ZrO₂,1 bis 30 Gew.-% sauerstoffhaltige Verbindungen des Kupfers, berechnet als CuO, 15 bis 50 Gew.-% sauerstoffhaltige Verbindungen des Nickels, berechnet als NiO, wobei das molare Ni : Cu-Verhältnis größer 1 ist, 15 bis 50 Gew.-% sauerstoffhaltige Verbindungen des Kobalts, berechnet als CoO, 0 bis 10 Gew.-% sauerstoffhaltige Verbindungen des Aluminiums und/oder Mangans, berechnet als Al₂O₃ bzw. MnO₂, und keine sauerstoffhaltigen Verbindungen des Molybdäns enthält, beispielsweise der in diesem Dokument offenbarte Katalysator A mit der Zusammensetzung 33 Gew.-% Zr, berechnet als ZrO₂, 28 Gew.-% Ni, berechnet als NiO, 11 Gew.-% Cu, berechnet als CuO und 28 Gew.-% Co, berechnet als CoO.

Weiterhin geeignet sind die in EP-A 696 572 offenbarten Katalysatoren, deren katalytisch aktive Masse vor der Reduktion mit Wasserstoff 20 bis 85 Gew.-% ZrO₂, 1 bis 30

Gew.-% sauerstoffhaltige Verbindungen des Kupfers, berechnet als CuO, 30 bis 70 Gew.-% sauerstoffhaltige Verbindungen des Nickels, berechnet als NiO, 0,1 bis 5 Gew.-% sauerstoffhaltige Verbindungen des Molybdäns, berechnet als MoO₃, und 0 bis 10 Gew.-% sauerstoffhaltige Verbindungen des Aluminiums und/oder Mangans, berechnet als Al₂O₃ bzw. MnO₂ enthält. Beispielsweise der in dieser Schrift konkret offenbarte Katalysator mit der Zusammensetzung 31,5 Gew.-% ZrO₂, 50 Gew.-% NiO, 17 Gew.-% CuO und 1,5 Gew.-% MoO₃. Ebenso geeignet sind die in WO-A-99/44984 beschriebenen Katalysatoren enthaltend (a) Eisen oder eine Verbindung auf der Basis von Eisen oder deren Gemische, (b) von 0,001 bis 0,3 Gew.-% bezogen auf (a) eines Promoters auf der Basis von 2, 3, 4 oder 5 Elementen ausgewählt aus der Gruppe Al, Si, Zr, Ti, V, (c) von 0 bis 0,3 Gew.-% bezogen auf (a) einer Verbindung auf der Basis eines Alkali- und/oder Erdalkalimetalls, sowie (d) von 0,001 bis 1 Gew.-% bezogen auf (a) Mangan.

Für Suspensionsverfahren werden bevorzugt Raney-Katalysatoren eingesetzt. Bei den Raney-Katalysatoren wird der aktive Katalysator als 'Metallschwamm' aus einer binären Legierung (Nickel, Eisen, Kobalt, mit Aluminium oder Silicium) durch Herauslösen eines Partners mit Säure oder Lauge hergestellt. Reste des ursprünglichen Legierungspartners wirken oft synergetisch.

Die im erfindungsgemäßen Verfahren eingesetzten Raney-Katalysatoren werden bevorzugt ausgehend von einer Legierung aus Kobalt oder Nickel, besonders bevorzugt Kobalt, und einer weiteren Legierungskomponente, die in Alkalien löslich ist, hergestellt. Bei dieser löslichen Legierungskomponente wird bevorzugt Aluminium verwendet, es können aber auch andere Komponenten wie Zink und Silicium oder Gemische aus solchen Komponenten eingesetzt werden.

Zur Aktivierung des Raney-Katalysators wird die lösliche Legierungskomponente ganz oder teilweise mit Alkali extrahiert, wofür zum Beispiel wässrige Natronlauge verwendet werden kann. Der Katalysator kann danach z. B. mit Wasser oder organischen Lösungsmittel gewaschen werden.

In dem Katalysator können einzelne oder mehrere weitere Elemente als Promotoren anwesend sein. Beispiele für Promotoren sind Metalle der Nebengruppen IB, VIB und/oder VIII des Periodensystems, wie Chrom, Eisen, Molybdän, Nickel, Kupfer usw.

Die Aktivierung der Katalysatoren durch Auslaugen der löslichen Komponente (typischerweise Aluminium) kann entweder im Reaktor selbst oder vor Einfüllen in den Reaktor erfolgen. Die voraktivierten Katalysatoren sind luftempfindlich und pyrophor und werden deshalb in der Regel unter einem Medium wie z. B. Wasser, einem organischen Lösungsmittel oder einem Stoff, der bei der erfindungsgemäßen Reaktion zugegen ist (Lösungsmittel, Edukt, Produkt) aufbewahrt und gehandhabt oder in eine organische Verbindung, die bei Raumtemperatur fest ist, eingebettet.

Bevorzugt wird erfindungsgemäß ein Kobalt-Skelett-Katalysator eingesetzt, der aus einer Co/Al-Legierung durch Laugung mit wässriger Alkalimetallhydroxid-Lösung, z.B. Natronlauge, und nachfolgender Waschung mit Wasser erhalten wurde, und bevorzugt als Promotoren mindestens eines der Elemente Fe, Ni, Cr enthält.

Solche Katalysatoren enthalten typischerweise neben Kobalt noch 1 - 30 Gew.-% Al, besonders 2 - 12 Gew.-% Al, ganz besonders 3 - 6 Gew.-% Al, 0 - 10 Gew.-% Cr, besonders 0,1 - 7 Gew.-% Cr, ganz besonders 0,5 - 5 Gew.-% Cr, insbesondere 1,5 - 3,5 Gew.-% Cr, 0 - 10 Gew.-% Fe, besonders 0,1 - 3 Gew.-% Fe, ganz besonders 0,2 - 1 Gew.-% Fe, und/oder 0 - 10 Gew.-% Ni, besonders 0,1 - 7 Gew.-% Ni, ganz besonders 0,5 - 5 Gew.-% Ni, insbesondere 1 - 4 Gew.-% Ni, wobei die Gewichtsangaben jeweils auf das Katalysatorgesamtgewicht bezogen sind.

Als Katalysator im erfindungsgemäßen Verfahren kann zum Beispiel vorteilhaft ein Kobalt-Skelett-Katalysator "Raney 2724" der Firma W. R. Grace & Co. eingesetzt werden. Dieser Katalysator weist folgende Zusammensetzung auf:
Al: 2-6 Gew.-%, Co: ≥86 Gew.-%, Fe: 0-1 Gew.-%, Ni: 1-4 Gew.-%, Cr: 1,5 - 3,5 Gew.-%.

Ebenfalls kann erfindungsgemäß ein Nickel-Skelett-Katalysator eingesetzt werden, der aus einer Ni/Al-Legierung durch Laugung mit wässriger Alkalimetallhydroxid-Lösung, z.B. Natronlauge, und nachfolgender Waschung mit Wasser erhalten wurde, und bevorzugt als Promotoren mindestens eines der Elemente Fe, Cr enthält. Solche Katalysatoren enthalten typischerweise neben Nickel noch
1 - 30 Gew.-% Al, besonders 2 - 20 Gew.-% Al, ganz besonders 5 - 14 Gew.-% Al,
0 - 10 Gew.-% Cr, besonders 0,1-7 Gew.-% Cr, ganz besonders 1 - 4 Gew.-% Cr, und/oder
0 - 10 Gew.-% Fe, besonders 0,1-7 Gew.-% Fe, ganz besonders 1 - 4 Gew.-% Fe, wobei die Gewichtsangaben jeweils auf das Katalysatorgesamtgewicht bezogen sind.

Als Katalysator im erfindungsgemäßen Verfahren kann zum Beispiel vorteilhaft ein Nickel-Skelett-Katalysator A 4000 der Firma Johnson Matthey eingesetzt werden. Dieser Katalysator weist folgende Zusammensetzung auf
Al≤ 14 Gew.-%, Ni: ≥ 80 Gew.-%, Fe: 1-4 Gew.-%, Cr: 1-4 Gew.-%.

Die Katalysatoren können gegebenenfalls bei nachlassender Aktivität und/oder Selektivität mit den dem Fachmann bekannten Methoden, wie zum Beispiel in WO 99/33561 und den darin zitierten Schriften veröffentlicht, regeneriert werden.

Die Regenerierung des Katalysators kann im eigentlichen Reaktor (in situ) oder am ausgebauten Katalysator (ex situ) durchgeführt werden. Bei Festbettverfahren wird bevorzugt in situ regeneriert, bei Suspensionsverfahren wird bevorzugt ein Teil des Katalysators kontinuierlich oder diskontinuierliche entnommen, ex situ regeneriert und zurückgeführt.

Die Temperaturen, bei denen das erfindungsgemäße Verfahren durchgeführt wird, liegen in einem Bereich von 40 bis 150°C, bevorzugt von 70 bis 140°C.

Der bei der Hydrierung herrschende Druck liegt im Allgemeinen bei 5 bis 300 bar, bevorzugt bei 30 bis 250 bar, besonders bevorzugt bei 40 bis 160 bar.

In einer bevorzugten Ausführungsform wird das Aminonitrilgemisch mit einer Rate der Hydrierung zugeführt, die nicht größer ist als die Rate, mit der das Aminonitrilgemisch mit Wasserstoff bei der Hydrierung reagiert.

Die Zuführrate ist bevorzugt so einzustellen, dass quasi Vollumsatz erreicht wird. Dieses wird durch Temperatur, Druck, Art des Gemisches, Menge und Art des Katalysators, des Reaktionsmediums, Durchmischungsgüte des Reaktorinhalts, Verweilzeit etc. beeinflusst.

Die optimalen Fahrbedingungen können sich bei der Hydrierung von einzelnen Aminonitrilen deutlich unterscheiden. Bei der erfindungsgemäßen Hydrierung eines Aminonitrilgemisches sind die einzustellenen Fahrbedingungen je nach Zusammensetzung jedoch nur leicht unterschiedlich und dadurch leichter optimierbar. Somit ist eine Flexibilität der eingesetzten Maschinen und Apparate nur in geringem Maße erforderlich, wie sie standardmäßig von Marktüblichen Geräten vorhanden ist (z.B. Förderleistung von Pumpen, Betriebstemperatur von Wärmeüberträgern, Druckauslegung der Apparate etc.).

Sofern im erfindungsgemäßen Verfahren ein Lösungsmittel verwendet wird, kann das Lösungsmittel zunächst vollständig mit dem Aminonitrilgemisch vermischt werden. Die erhaltene Lösung, die gegebenenfalls auch Wasser sowie weitere Additive enthalten kann, wird anschließend in das den Katalysator enthaltende Reaktionsgefäß zugeführt. Gegebenenfalls kann, beispielsweise bei Semibatch-Verfahren, ein Teil des Lösungsmittels zusammen mit dem Katalysator im Reaktionsgefäß vorgelegt werden, worauf die Lösung zudosiert wird. Bei kontinuierlichen Verfahren kann eine Teilmenge des Lösungsmittels auch separat von der Lösung, die das Aminonitrilgemisch, das Lösungsmittel und gegebenenfalls Wasser enthält, in das Reaktionsgefäß zugegeben werden. Da ein AAN/I DAN-Gemisch eingesetzt wird, ist auch eine komplett separate Zudosierung des Lösungsmittels denkbar.

Das erfindungsgemäße Verfahren zur Herstellung von Ethylenaminen durch Hydrierung von Aminonitrilgemischen kann in üblichen für die Katalyse geeigneten Reaktionsgefäßen in einer Festbett-, Wirbelschicht-, Suspensionsfahrweise kontinuierlich, semikontinuierlich oder diskontinuierlich durchgeführt werden. Zur Durchführung der Hydrierung eignen sich Reaktionsgefäße, in denen eine Kontaktierung des Aminonitrilgemisches und des Katalysators mit dem gasförmigen Wasserstoff unter Druck möglich ist.

Die Hydrierung in Suspensionsfahrweise kann in einem Rührreaktor, Strahlschlaufenreaktor, Strahldüsenreaktor, Blasensäulenreaktor, bzw. in einer Kaskade derartiger gleicher oder verschiedener Reaktoren durchgeführt werden. Für die Hydrierung an einem Festbettkatalysator sind Rohrreaktoren aber auch Rohrbündelreaktoren denkbar.

Im Fall eines Festbettkatalysators wird in Sumpf- oder Rieselfahrweise mit dem Aminonitrilgemisch beaufschlagt. Bevorzugt wird allerdings die Suspensionsfahrweise in semikontinuierlicher und bevorzugt in kontinuierlicher Fahrweise eingesetzt.

Die Hydrierung der Nitrilgruppen findet unter Freisetzung von Wärme statt, die in der Regel abgeführt werden muss. Die Wärmeabfuhr kann durch eingebaute Wärmeübertragerflächen, Kühlmantel oder außenliegende Wärmeübertrager in einem Umlaufkreis um den Reaktor erfolgen. Der Hydrierreaktor bzw. eine Hydrierreaktorkaskade kann in geradem Durchgang gefahren werden. Alternativ ist auch eine Kreislauffahrweise möglich, bei der ein Teil des Reaktoraustrages an den Reaktoreingang zurückgeführt wird, bevorzugt ohne vorherige Aufarbeitung des Kreislaufstromes. Damit lässt sich eine optimale Verdünnung der Reaktionslösung erreichen. Insbesondere kann der Kreislaufstrom mittels eines externen Wärmeübertragers auf einfache und kostengünstige Weise gekühlt und somit die Reaktionswärme abgeführt werden. Der Reaktor lässt sich dadurch auch adiabat betreiben, wobei der Temperaturanstieg der Reaktionslösung durch den gekühlten Kreislaufstrom begrenzt werden kann. Da der Reaktor selbst dann nicht gekühlt werden muss, ist eine einfache und kostengünstige Bauform möglich. Eine Alternative stellt ein gekühlter Rohrbündelreaktor (nur im Fall des Festbetts) dar. Auch eine Kombination der beiden Fahrweisen ist denkbar. Hierbei wird bevorzugt ein Festbett- einem Suspensionsreaktor nachgeschaltet.

Das erfindungsgemäße Verfahren liefert ein Ethylenamingemisch, das als Hauptkomponente EDA und DETA sowie als Nebenkomponenten weitere Ethylenamine (z. B. Piperazin) enthält. Das Verhältnis der Edukte AAN und IDAN findet sich prinzipiell nach der Hydrierung bei den entsprechenden Produkten EDA und DETA wieder. Je nach Hydrierbedingungen kann aus AAN weiteres DETA gebildet werden. Dadurch kann der DETA-Anteil des resultierenden Amingemisches, das als hauptsächlichen Bestandteil EDA enthält, um 1-10 Gew.% steigen.

Im Anschluss an die Hydrierung kann das erhaltene Produkt (Ethylenamingemisch) gegebenenfalls weiter aufgereinigt werden, beispielsweise indem das gegebenenfalls verwendete Lösungsmittel, Wasser und/oder der Katalysator nach dem Fachmann bekannten Methoden abgetrennt werden. Insbesondere können die beiden Hauptprodukte (EDA und DETA) gemeinsam oder einzeln nach dem Fachmann bekannten Methoden aus dem Ethylenamingemisch isoliert werden. Sofern die beiden Hauptprodukte gemeinsam isoliert werden, beispielsweise durch eine Destillation, können sie anschließend in die beiden Einzelprodukte isoliert werden. Letztendlich erhält man somit reines EDA und reines DETA. Sonstige Verunreinigungen, Nebenprodukte oder weitere Ethylenamine können ebenfalls mit dem Fachmann bekannten Methoden aus dem Ethylenamingemisch abgetrennt werden.

In einer bevorzugten Ausführungsform wird das erfindungsgemäße Verfahren unter Verwendung von Tetrahydrofuran oder Methanol als Lösungsmittel durchgeführt. Die Temperatur bei der Hydrierung beträgt vorzugsweise 80 bis 140°C, der Druck vorzugsweise 40 bis 160 bar. Vorzugsweise wird die Hydrierung in Abwesenheit von Ammoniak durchgeführt.

Mit dem erfindungsgemäßen Verfahren wird eine hohe Katalysatorbelastung erzielt, die ein Maß für die Aktivität des verwendeten Katalysators ist. Bevorzugt beträgt die Katalysatorbelastung 0,3 bis 20 mol Nitril (entspricht ~0,2g bis 12g AAN/g Kat), vorzugsweise 1 bis 10 mol Nitril (-0,6g-6g) pro Gramm Katalysator pro Stunde. Je höher die Katalysatorbelastung ist, umso höher kann auch die Raum-Zeit-Ausbeute an Ethylenaminen sein.

Die nachfolgenden Beispiele verdeutlichen das erfindungsgemäße Verfahren. Die Anteile sind in Gew.-% angegeben, sofern nicht anders aufgeführt. Ein mitgeführter interner Standard, Diethylenglykoldimethylether (DEGDME), erlaubt eine Quantifizierung des Produktes durch Bestimmung der eventuell gebildeten flüchtigen Zersetzungsbestandteile. Die Quantifizierung erfolgt mittels Gaschromatographie (GC), wobei den jeweils entnommenen Proben zur Homogenisierung Methanol zugegeben wird.

### Beispiele

### Beispiel 1 (kontinuierliche Hydrierung / 30 Gew.-% Wasser)

In einen 270ml-Autoklaven mit Stromstörern und Scheibenrührer werden 10g Cr-dotierter Raney-Kobalt vorgelegt und kontinuierlich 50NI (Normliter)/h Wasserstoff zugefahren. Eine Mischung aus 30g AAN, 9g Wasser in 255g THF wird kontinuierlich pro Stunde bei 50 bar zugepumpt. Über eine Tauchfritte wird kontinuierlich Reaktionsgemisch ausgetragen. Die Reaktionstemperatur wird auf 120°C gehalten. Der Austrag wird über ein Regelventil entspannt. Regelmäßige Proben werden mittels GC analysiert. Zu keiner Zeit kann AAN im Austrag nachgewiesen werden. Die Proben zeigen gleich bleibend eine Selektivität von > 98 % EDA sowie 1% DETA.

Anschließend werden für 7h 6g des AAN durch 10g IDAN ersetzt, also pro Stunde 24g AAN, 10g IDAN sowie 255g THF zugepumpt. In den GC-Analysen kann kein Nitril mehr nachgewiesen werden. Hierbei werden Selektivitäten von 66 % EDA, 30 % DETA sowie 1 % Piperazin erzielt.

Für weitere 7h werden pro Stunde neben 18g AAN (0,32mol) zusätzlich 22,5g IDAN in 255g THF inkl. 24g Wasser zudosiert. Auch in diesem Fall liegt Vollumsatz von AAN und IDAN vor. Die Selektivitäten des Gemischs liegen bei 41 % EDA, 51 % DETA sowie 3% Piperazin.

### Vergleichsbeispiel 1: Kontinuierliche Hydrierung von kristallisiertem IDAN (wasserfrei)

### A) Standard:

In einen 270ml-Autoklav mit Stromstörern und Scheibenrührer wird 10 g Cr-dotierter Raney-Kobalt vorgelegt und kontinuierlich 50NI/h Wasserstoff zugefahren. Eine Mischung aus 2,9 g IDAN in 60 g THF wird kontinuierlich pro Stunde bei 180 bar zugepumpt. Über eine Tauchfritte wird kontinuierlich Reaktionsgemisch ausgetragen. Die Reaktionstemperatur wird auf 120°C gehalten. Der Austrag wird über ein Regelventil entspannt. Regelmäßige Proben werden mittels GC analysiert. Während der Versuchsdauer von 140 h kann kein IDAN nachgewiesen werden. Die Selektivitäten liegen bei 0,5 % EDA, 90 % DETA sowie 4 % Piperazin.

### B) Höhere Belastung

In einen 270 ml-Autoklav mit Stromstörern und Scheibenrührer werden 6 g Cr-dotierter Raney-Kobalt vorgelegt und kontinuierlich 50NI/h Wasserstoff zugefahren. Eine Mischung aus 7,5 g IDAN in 140 g THF wird kontinuierlich pro Stunde bei 170 bar zugepumpt. Über eine Deckelfritte wird kontinuierlich Reaktionsgemisch ausgetragen. Die Reaktionstemperatur wird auf 120°C gehalten. Der Austrag wird über ein Regelventil entspannt. Regelmäßige Proben werden mittels GC analysiert. Nach 9 h können 4 % IDAN nachgewiesen werden. Die Selektivität liegt bei lediglich 68 % bezüglich DETA. Nach 24 h kann nur noch 16 % DETA bei 40 % Umsatz nachgewiesen werden.

Die vorstehenden Beispiele zeigen, dass im erfindungsgemäßen Verfahren das im Aminonitrilgemisch vorliegende IDAN deutlich schneller hydriert werden kann als in Verfahren nach dem Stand der Technik (Vergleichsbeispiele). Trotz der Gegenwart von AAN kann somit in einer Stunde die 30-fache Menge an IDAN hydriert werden im Vergleich zur konventionellen IDAN-Hydrierung. Weiterhin ist festzustellen, dass IDAN auch bei viel niedrigeren Drücken hydriert werden kann. Dies wirkt sich vorteilhaft auf die verwendeten Gerätschaften aus, zudem kann das erfindungsgemäße Verfahren in der gleichen Apparatur durchgeführt werden wie die konventionelle Einzelhydrierung von AAN zu EDA.

## Patentansprüche

1. Verfahren zur Herstellung eines Ethylenamingemisches, wobei ein Aminonitrilgemisch enthaltend mindestens 30 Gew.-% Aminoacetonitril (AAN) und mindestens 5 Gew.-% Iminodiacetonitril (IDAN) in Gegenwart eines Katalysators hydriert wird.

2. Verfahren gemäß Anspruch 1, **dadurch gekennzeichnet, dass** ein Raney-Katalysator, insbesondere ein Raney-Nickel- oder ein Raney-Kobalt-Katalysator eingesetzt wird.

3. Verfahren gemäß Anspruch 1 oder 2, **dadurch gekennzeichnet, dass** die Hydrierung in Gegenwart von Wasser und/oder einem organischen Lösungsmittel, insbesondere Tetrahydrofuran oder Methanol, durchgeführt wird.

4. Verfahren gemäß einem der Ansprüche 1 bis 3, **dadurch gekennzeichnet, dass** der Druck 40 bis 160 bar und/oder die Temperatur 80°C bis 140°C beträgt.

5. Verfahren gemäß einem der Ansprüche 1 bis 4, **dadurch gekennzeichnet, dass** im Aminonitrilgemisch 10 bis 25 Gew.-% IDAN enthalten ist.

6. Verfahren gemäß einem der Ansprüche 1 bis 5, **dadurch gekennzeichnet, dass** nach der Hydrierung Ethylendiamin (EDA) und Diethylentriamin (DETA) und gegebenenfalls weitere Ethylenamine aus dem Ethylenamingemisch isoliert werden.

7. Verfahren gemäß einem der Ansprüche 1 bis 6, **dadurch gekennzeichnet, dass** AAN und/oder IDAN durch Umsetzung von NH₃ und Formaldehydcyanhydrin (FACH) hergestellt werden.

8. Verfahren gemäß einem der Ansprüche 1 bis 7, **dadurch gekennzeichnet, dass** das Aminonitrilgemisch mit einer Rate der Hydrierung zugeführt wird, die nicht größer ist als die Rate, mit der das Aminonitrilgemisch mit Wasserstoff bei der Hydrierung reagiert.

9. Verfahren gemäß einem der Ansprüche 1 bis 8, **dadurch gekennzeichnet, dass** die Hydrierung ohne Zusatz von Ammoniak durchgeführt wird.

## Claims

1. A process for preparing an ethylene amine mixture, which comprises hydrogenating an amino nitrile mixture comprising at least 30% by weight of aminoacetonitrile (AAN) and at least 5% by weight of iminodiacetonitrile (IDAN) in the presence of a catalyst.

2. The process according to claim 1, wherein a Raney catalyst, in particular a Raney nickel catalyst or a Raney cobalt catalyst, is used.

3. The process according to claim 1 or 2, wherein the hydrogenation is carried out in the presence of water and/or an organic solvent, in particular tetrahydrofuran or methanol.

4. The process according to any of claims 1 to 3, wherein the pressure is from 40 to 160 bar and/or the temperature is from 80°C to 140°C.

5. The process according to any of claims 1 to 4, wherein the aminonitrile mixture comprises from 10 to 25% by weight of IDAN.

6. The process according to any of claims 1 to 5, wherein ethylenediamine (EDA) and diethylenetriamine (DETA) and optionally further ethylene amines are isolated from the ethylene amine mixture after the hydrogenation.

7. The process according to any of claims 1 to 6, wherein AAN and/or IDAN are prepared by reaction of NH₃ and formaldehyde cyanohydrins (FACH).

8. The process according to any of claims 1 to 7, wherein the amino nitrile mixture is fed into the hydrogenation at a rate which is no greater than the rate at which the amino nitrile mixture reacts with hydrogen in the hydrogenation.

9. The process according to any of claims 1 to 8, wherein the hydrogenation is carried out without addition of ammonia.

## Revendications

1. Procédé pour la préparation d'un mélange d'éthylène-amines, où un mélange d'aminonitriles contenant au moins 30% en poids d'aminoacétonitrile (AAN) et au moins 5% en poids d'iminodiacétonitrile (IDAN) est hydrogéné en présence d'un catalyseur.

2. Procédé selon la revendication 1, **caractérisé en ce qu'**on utilise un catalyseur de Raney, en particulier un catalyseur de Raney à base de nickel ou un catalyseur de Raney à base de cobalt.

3. Procédé selon la revendication 1 ou 2, **caractérisé en ce que** l'hydrogénation est réalisée en présence d'eau et/ou d'un solvant organique, en particulier le tétrahydrofuranne ou le méthanol.

4. Procédé selon l'une quelconque des revendications 1 à 3, **caractérisé en ce que** la pression est de 40 à 160 bars et/ou la température est de 80°C à 140°C.

5. Procédé selon l'une quelconque des revendications 1 à 4, **caractérisé en ce que** 10 à 25% en poids d'IDAN sont contenus dans le mélange d'aminonitriles.

6. Procédé selon l'une quelconque des revendications 1 à 5, **caractérisé en ce qu'**après l'hydrogénation, de l'éthylènediamine (EDA) et de la diéthylènetriamine (DETA) et le cas échéant d'autres éthylène-amines sont isolées à partir du mélange d'éthylène-amines.

7. Procédé selon l'une quelconque des revendications 1 à 6, **caractérisé en ce que** l'AAN et/ou l'IDAN sont préparés par transformation de NH₃ et de formaldéhyde-cyanhydrine (FACH).

8. Procédé selon l'une quelconque des revendications 1 à 7, **caractérisé en ce que** le mélange d'aminonitriles est ajouté à l'hydrogénation à une vitesse qui n'est pas supérieure à la vitesse à laquelle le mélange d'aminonitriles réagit avec l'hydrogène lors de l'hydrogénation.

9. Procédé selon l'une quelconque des revendications 1 à 8, **caractérisé en ce que** l'hydrogénation est réalisée sans addition d'ammoniac.
